# EUROPÄISCHE PATENTSCHRIFT

(11) **EP 3 048 967 B1**
(45) Veröffentlichungstag und Bekanntmachung des Hinweises auf die Patenterteilung: **06.02.2019**
(21) Anmeldenummer: 14749708.5
(22) Anmeldetag: 24.06.2014
(51) Int. Cl.: A61B 5/053, A61B 5/00

(54) **VERFAHREN UND VORRICHTUNG ZUR BIOELEKTRISCHEN IMPEDANZ-MESSUNG (BIA) DES KÖRPERS EINER PERSON**
METHOD AND DEVICE FOR A BIOELECTRIC IMPEDANCE ANALYSIS (BIA) OF THE BODY OF A PERSON
PROCÉDÉ ET DISPOSITIF DE MESURE D'IMPÉDANCE BIOÉLECTRIQUE (BIA) DE L'ORGANISME D'UNE PERSONNE

(30) Priorität: 26.06.2013 DE 102013106690
(43) Veröffentlichungstag der Anmeldung: 03.08.2016
(73) Patentinhaber: TOMCZAK, Jörg, 83125 Eggstätt (DE)
(72) Erfinder: TOMCZAK, Jörg, 83125 Eggstätt (DE)
(74) Vertreter: dompatent von Kreisler Selting Werner - Partnerschaft von Patent- und Rechtsanwälten mbB
(86) Internationale Anmeldenummer: PCT/DE2014/000318
(87) Internationale Veröffentlichungsnummer: WO 2014/206391

(56) Entgegenhaltungen:
- US-A1- 2009 182 243
- US-A1- 2013 006 086
- US-B1- 6 256 532

## Beschreibung

Die Erfindung betrifft ein Verfahren und eine Vorrichtung zur bioelektrischen Impedanz-Messung (BIA) des Körpers einer Person, wobei mehrere einen Wechselstrom führenden Elektrodenpaare (hier: Eingangselektroden) und mehrere den Spannungsabfall messende sensitive Elektrodenpaare (hier: Ausgangselektroden) mit der Oberfläche des Körpers in Kontakt gebracht und zwischen diesen Ausgangselektroden die bioelektrische Impedanz der Körperabschnitte messtechnisch erfasst und die erhaltenen Messwerte mit gespeicherten, beispielsweise statistischen Daten, zu einer Körperstruktur - und viszeralen Risikoanalyse umgerechnet werden.

Während der Messung erzeugt ein in einer Messvorrichtung generierter Wechselstrom im kHz-Bereich über die Eingangselektroden im Körper der Person ein elektromagnetisches Feld, das sich zwischen den Eingangselektroden in allen Gewebeschichten dreidimensional ausbreitet und mit den Ausgangselektroden gemessen wird. Um bei der Messung einen möglichst aussagefähigen Körperbereich zu erfassen, werden die Elektroden an charakteristischen markanten Stellen des Körpers platziert und mit einer Messvorrichtung verbunden.

Um eine zum Körperfett in Beziehung stehende Größe zu bestimmen, ist eine Hand-Fuß-Messung der inneren Impedanz des Körpers beispielsweise schon mit nur vier Elektroden möglich. Hierbei wird der Person jeweils eine Eingangselektrode am rechten Fuß und an der rechten Hand und in deren Nähe jeweils eine Ausgangselektrode befestigt, so dass während der Messung ein elektromagnetisches Feld zwischen den Impuls gebenden Elektroden der rechten Hand und dem rechten Fuß aufgebaut wird. Die gemessene Potentialdifferenz zwischen den Ausgangselektroden sowie die Stärke des durchgehenden Stroms und die Phasenverschiebung stellen dann ein Maß für die Impedanz des Körpers dar.

In der DE 602 05 976 T2 wird zur Beurteilung bzw. zur Abschätzung des viszeralen Körperfettverhältnisses ein Messgerät mit acht Elektroden beschrieben, mit dem neben einer Hand-Hand- und Fuß-Fuß-Messung der bioelektrischen Impedanz auch das Körpergewicht sowie Blutdruck und Pulsfrequenz der Person gemessen werden können. Hierzu ist das Messgerät in Form einer L-förmigen Waage ausgebildet, auf dessen Fußteil vier Fußelektroden und an dessen seitlichen Handgriffen jeweils zwei Handelektroden angeordnet sind. Zur Messung stellt sich die Person so auf das Fußteil, dass jeweils eine Zehe des rechten und des linken Fußes Kontakt mit der Eingangselektrode und die Ferse des rechten und des linken Fußes Kontakt mit der Ausgangselektrode hat. Gleichzeitig werden beide seitlichen Handgriffe mit den Händen angefasst, wodurch der Kontakt mit den dort angeordneten Ein- und Ausgangs-Handelektroden hergestellt wird.

Um den Anteil unterschiedlicher Körperfettmassen durch Bestimmung der bioelektrischen Impedanz eines Körpers zu berechnen, wird in der DE 11 2008 001 501 T5 eine Fuß-Fuß- und Hand-Hand-, kombiniert mit einer Bauch-Messung mit insgesamt zwölf Elektroden vorgeschlagen. Zur Messung werden der Person in Liegeposition mit einer Befestigungseinheit in Form eines Gürtels vier Elektroden - zwei Ein- und zwei Ausgangselektroden am Bauch sowie mittels Manschetten jeweils eine Ein- und Ausgangselektrode an den oberen Gliedmaßen, hier an den Handgelenken und jeweils eine Ein- und Ausgangselektrode an den unteren Gliedmaßen, hier an den Fußknöcheln befestigt. Nachteilig bei bekannten Methoden zur Messung der bioelektrischen Impedanz eines Körpers ist der oftmals beträchtliche Aufwand der Verkabelung, insbesondere bei Einbeziehung einer Bauch-Messung, nämlich dass eine Person eine solche Messung nicht eigenständig bzw. doch nur sehr schwierig durchführen kann. Zudem ist gefordert, dass diese Person ohne Schuhe und sonstige Beinbekleidung, wie etwa auch Stützstrümpfe sein muss, um dann liegend gemessen werden zu können.

US 2009/0182243 beschreibt eine Vorrichtung zur bioelektrischen Impedanz-Messung. Hierzu weist die Vorrichtung eine Fußfläche mit Eingangs- und Ausgangselektroden auf sowie ein damit verbundenes Handteil. Das Handteil weist eine Fläche auf, die gegen den Bauch gehalten wird. Hierzu sind Griffe für die linke und die rechte Hand vorgesehen. Dabei weisen die Griffe und auch die Fläche des Handteils mehrere Eingangs- und Ausgangselektroden auf.

Ausgehend vom geschilderten Stand der Technik ist es Aufgabe der Erfindung, eine Messvorrichtung anzugeben, mit der von einer Person in einfacher Weise gleichzeitig eine BIA-Bauch, eine BIA-Bauch-Hand und eine BIA-Hand-Hand selbständig durchgeführt werden kann.

Die gestellte Aufgabe wird verfahrensmäßig mit den kennzeichnenden Merkmalen des Anspruchs 1 und vorrichtungsmäßig mit den kennzeichnenden Merkmalen des Anspruchs 5 dadurch gelöst, dass mittels einer bandförmig ausgebildeten Messschablone, die mit einem Messmodul verbunden ist und die in den beiden mittleren Schablonenabschnitten jeweils links und rechts eine Eingangselektrode und eine Ausgangselektrode und an den Handgriffen der äußeren Schablonenabschnitte jeweils links und rechts eine Eingangselektrode und eine Ausgangselektrode aufweist, von der Person autark in einfacher Weise gleichzeitig eine BIA-Bauch, eine BIA-Bauch-Hand und eine BIA-Hand-Hand durchgeführt wird, wozu die Messschablone von der Person an den Griffen - mit Daumen-Zeigefingerkontakt an den dortigen Elektroden - gehalten und ihre mittleren Schablonenabschnitte mit den dort angeordneten Elektroden in
definierter Position in beliebiger Körperhaltung stehend, sitzend oder liegend am Bauch angelegt werden.

Zur Herstellung der definierten Position ist die Messschablone so ergonomisch geformt, dass die Messung mit einer vorgegebenen Armhaltung durchgeführt werden muß. Damit die Messung auch von unterschiedlich dicken Personen durchgeführt werden kann, sind nach einer vorteilhaften Ausbildung der Erfindung die äußeren Schablonenabschnitte der Messschablone so elastisch ausgebildet, dass sie sich während des Messvorgangs dem Bauchumfang der Person anpassen lassen.

Die Schablonenmitte ist mit einer Markierung, beispielsweise einer Aussparung gekennzeichnet. Zur Messung wird diese Markierung an den Bauchnabel angelegt und die an der Unterseite der Messschablone angeordneten Bauchelektroden gegen den Bauch gehalten. Hierdurch ist sichergestellt, dass sich die Bauchelektroden bei jeder Messung in gleicher Positionierung seitlich vom Bauchnabel befinden. Zweckmäßigerweise werden die Elektroden mit einem Tröpfchen Elektrodengel benetzt.

Die Bauchelektroden sind symmetrisch so um die Markierung angeordnet, dass sich die Ausgangselektroden beispielsweise links und rechts 7 cm und weiter außen sich die Eingangselektroden beispielsweise links und rechts 11 cm entfernt von der Markierung befinden.

Mit Vorteil sind die bei bekannten Messvorrichtungen für die Messung sonst erforderlichen oftmals komplizierten Befestigungseinheiten und Verkabelungen für die Elektroden somit nicht erforderlich.

Die bei einer Messfrequenz von beispielsweise 50 kHz Wechselstromimpulsen erzeugten Messwerte werden durch das Messmodul erfasst und können mit einer verbundenen Computereinheit bzw. Berechnungseinheit zur Erstellung einer Körperstruktur- und viszeralen Risikoanalyse verwendet werden. Erfindungsgemäß ist es aber auch möglich, die Messung mit einer weiteren und/oder mehreren Messfrequenzen zwischen 1-1000 kHz Wechselstromimpulsen durchzuführen.

Das Messmodul, das zur Durchführung der Messung einen Stromimpulserzeuger für die Eingangselektroden und einen Spannungsabfallmesser für die Ausgangselektroden enthält, ist erfindungsgemäß als fester Bestandteil in die Messschablone integriert. Alternativ ist das Messmodul als separates Gerät ausgebildet und mit der Messschablone über eine entsprechende Vorrichtung verbunden.

Beispielsweise ist das Messmodul in eine an der Oberseite der Messschablone angeordnete Kontaktstiftleiste eingeschoben, in die die Verbindungsleitungen der Elektroden einmünden, oder das Messmodul ist über eine die ElektrodenVerbindungsleitungen enthaltende gemeinsame Leitung mit der Messschablone verbunden oder das integrierte Messmodul sendet die Messdaten per Funk an eine externe Auswertungseinheit.

Die mit Hilfe der Messschablone durchgeführten Messungen werden zur Erstellung einer viszeralen Risikoanalyse verwendet. Die BIA-Bauch sagt aus: je größer der gemessene Widerstand (dabei unerheblich ob Resistanz oder Impedanz) desto größer ist das viszerale Risiko. Etwa 50 % des Widerstandes generiert dabei das Unterhautfettgewebe. Die zweite Hälfte teilen sich die weiteren Bestandteile des Bauchraumes, wie Flüssigkeiten (intra- und extrazelluläre), Bauchmuskulatur, intramuskuläre Fetteinlagerungen, Bauchorgane, viszerale Fetteinlagerungen, Knochen bzw. Wirbelsäule. Zusammengefasst gibt mit hoher Aussagequalität die reine BIA-Bauchmessung die Schichtendicke des Unterhautfettgewebes und darüber hinaus eine Aussage über weitere interne Bauchkompartimente an.

Die BIA-Bauch-Hand und BIA-Hand-Hand stellen ebenso keine reine Fettmessung, sondern Körperstrukturanalysen insbesondere in bezug auf die Muskel/Fett-Relation dar. Durch die Kombination von BIA-Bauch und BIA-Bauch-Hand und BIA-Hand-Hand lassen sich in bezug auf eine viszerale Risikoanalyse bisher bestehende Unsicherheiten weitgehend verkleinern und als Körperstrukturanalyse erweitern.

Das erfindungsgemäße Verfahren verwendet vier stromgebende Eingangselektroden (beide Zeigefinger und beide äußeren Bauchelektroden) der Messschablone, die zu sechs möglichen Paar-Verbindungen miteinander kombiniert werden können. Ebenso gibt es an der Messschablone vier Ausgangselektroden, die ebenso zu sechs möglichen Paar-Verbindungen kombinierbar sind. Insgesamt sind also 36 Messstrecken möglich, die theoretisch nacheinander durchgemessen werden können. Real sind jedoch nur einige dieser Messstrecken für eine sinnvolle Berechnung notwendig.

Weitere Einzelheiten und Vorteile der Erfindung werden nachfolgend an Hand von in schematischen Zeichnungsfiguren dargestellten Ausführungsbeispielen näher erläutert.

Es zeigen:
- Figur 1: mögliche Verbindungen und elektromagnetische Feldlinien zwischen vier Eingangs-Elektroden,
- Figur 2 - 5: Messanordnungen für unterschiedliche Körperbereiche,
- Figur 6: eine Messschablone der Erfindung in einer perspektivischen Vorderansicht.

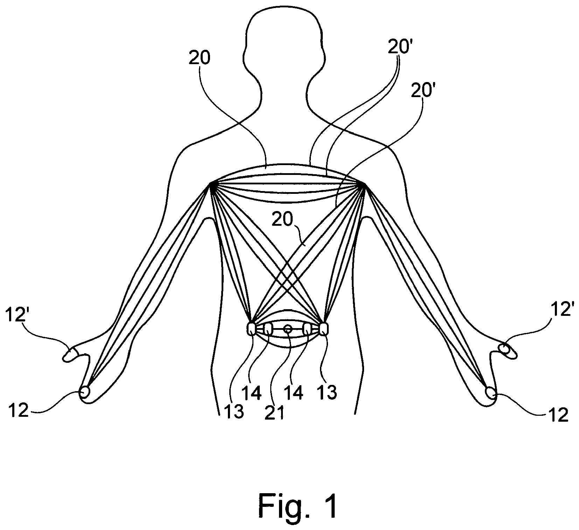

In der Figur 1 ist in einer Vorderansicht der Oberkörper einer Person dargestellt. In beiden Händen befindet sich im Bereich des Zeigefingers eine Eingangselektrode 12 und im Bereich des Daumens eine Ausgangselektrode 12'. Weiterhin befindet sich rechts und links vom Bauchnabel 21 je eine Ausgangselektrode 14 und daneben nach außen versetzt rechts und links vom Bauchnabel 21 je eine Eingangselektrode 13.

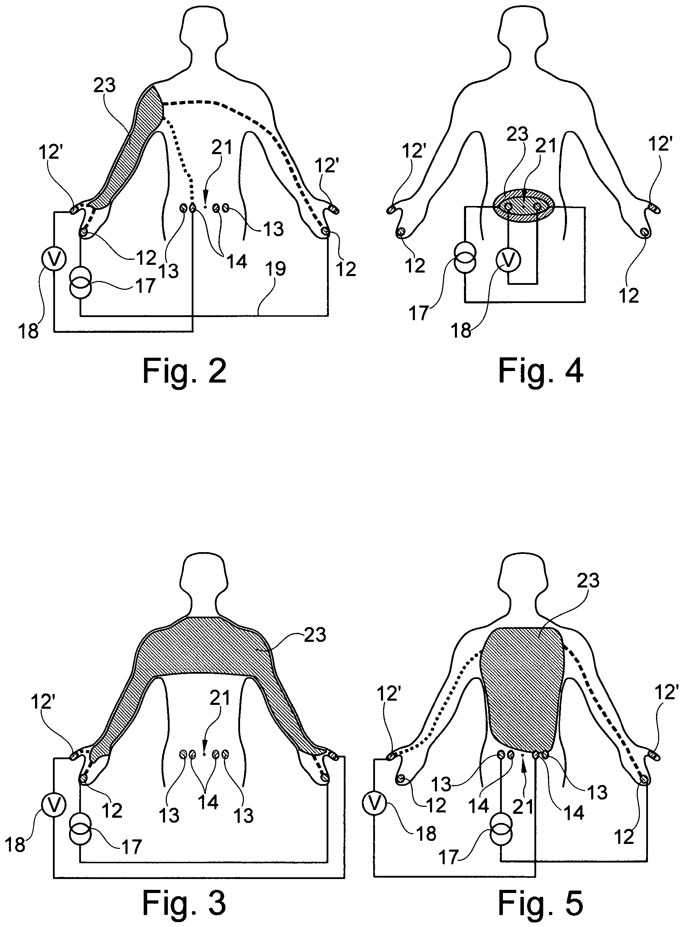

Die bei einer entsprechenden Aktivierung zwischen den miteinander durch Elektrodenverbindungsleitungen (19, Fig. 2) mit einem zwischengeschalteten Stromimpulserzeuger (17, Fig. 2 - 5) verbundenen Eingangselektroden 12 und 13 sich ergebenden Feldlinien 20', bzw. die erzeugten elektromagnetischen Felder 20 sind entsprechend in den Oberkörper der Person eingezeichnet.

Gemessen werden diese elektromagnetischen Felder 20 bzw. ihre Abschwächung durch die über Elektrodenverbindungsleitungen (19, Fig. 2) mit einem zwischengeschalteten Spannungsabfallmesser (18, Fig. 2 - 5) miteinander verbundenen Ausgangselektroden 12' und 14.

Wie der schematischen Darstellung der Figur 1 deutlich zu entnehmen ist, lässt sich allein durch nur vier Eingangselektroden fast im gesamten Oberkörper und Bauchraum nebst Armen der Person ein messtechnisch relevantes Feld generieren. Durch wahlweise Kombination einzelner Elektrodenpaare lassen sich ganz gezielt einzelne Messbereiche 23 des Körpers messtechnisch erfassen, wie in den nachfolgenden Zeichnungsfiguren 2 bis 5 beispielhaft schematisch dargestellt ist.

In der Figur 2 ist ein über den rechten Arm sich erstreckender dunkel hervorgehobener Messbereich 23 eingezeichnet, der messtechnisch erfasst werden soll. Zur Realisierung der Messung werden die Eingangselektroden 12 des linken und des rechten Zeigefingers aktiviert und die Ausgangselektroden 12' des rechten Daumens der rechten Ausgangselektrode 14 der Bauchelektroden messen den Spannungsabfall und die Phasenverschiebung dieses Feldes ab.

In der Figur 3 wird mit identischen Eingangselektroden 12 der beiden Zeigefinger der Fig. 2 eine Änderung der Ausgangselektroden vorgenommen. Die Ausgangselektrode 12' des linken und rechten Daumens bestimmen den Messbereich, den in der Fig. 3 dunkel hervorgehoben Messbereich 23 der Schulter, des oberen Brustkorbes und der beiden Arme (BIA-Hand-Hand). Die Bauchelektroden 13, 14 wurden bei diesem Beispiel nicht in die Messung einbezogen.

In der Figur 4 ist eine ausschließliche Messung des Unterbauchs (BIA-Bauch) entsprechend dem dunkel hervorgehobenen Messbereich 23 durchzuführen. Hierzu werden ausschließlich die Bauchelektroden 13, 14 bei dieser Messung verwendet, wobei die beiden äußeren Eingangselektroden 13 das Feld generieren und die beiden inneren Ausgangselektroden 14 den Spannungsabfall und die Phasenverschiebung abmessen. Bei diesem Beispiel sind also die Handelektronen nicht in die Messung einbezogen.

Eine Ausmessung des gesamten Oberkörpers (BIA-Bauch-Hand), in der Figur 5 als dunkel hervorgehobener Messbereich 23 dargestellt, ist mit folgender Elektrodenkombination möglich: Als Eingangselektroden fungieren 12 des linken Zeigefingers und 13 der rechten Bauchelektrode. Ausgangselektroden sind 12' des rechten Daumens und die rechte der Bauchelektroden 14.

Insbesondere die Elektrodenkombinationen der Figuren 2 und 5, in dernen Bauchelektroden und Handelektroden gleichzeitig verwendet werden, sind in einfacher Weise mit einer erfindungsgemäßen Messschablone durchführbar und ergänzen sich in den Aussagequalitäten über das medizinisch relevante "viszerale Risiko".

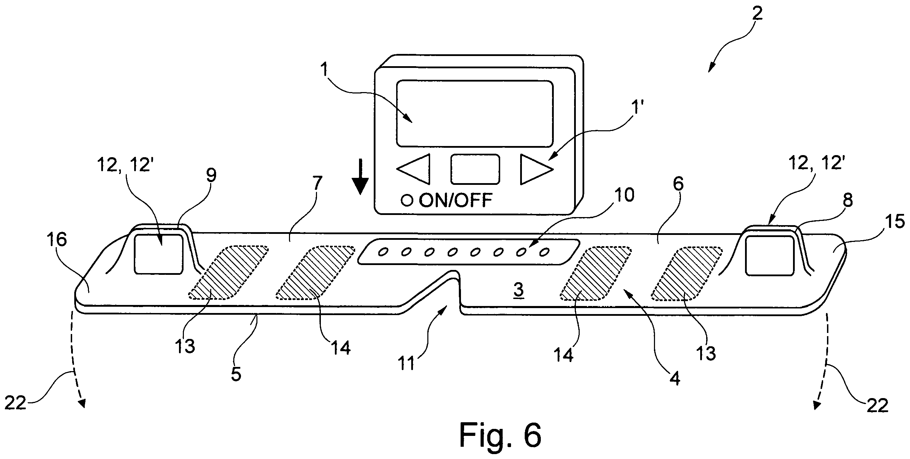

In der Figur 6 ist in einer perspektivischen Vorderansicht schematisch der Aufbau einer derartigen Messschablone 2 als Skizze dargestellt. Die in Form der Lenkstange eines Fahrradlenkers ausgebildete Messschablone 2 besteht aus einem ergonomisch geformten bandförmigen Grundkörper 3, der auf seiner Oberseite 4 im Bereich seiner äußeren Schablonenabschnitte 15, 16 links und rechts mit Handgriffen 8, 9 versehen ist. Mit diesen Handgriffen 8, 9 kann die Messschablone 2 wegen ihrer ergonomischen Form nur in einer vorgegebenen Armhaltung gehalten und am eigenen Bauch angelegt werden. Zur Unterstützung einer reproduzierbaren am Bauch anliegenden Halteposition sind die äußeren Schablonenabschnitte 15, 16 elastisch ausgebildet, so dass sie sich in Pfeilrichtung 22 verbiegen und beim Halten der Messschablone 2 dem Bauchumfang anpassen können.

In jedem der Handgriffe 8, 9 befindet sich eine Eingangselektrode 12 und eine Ausgangselektrode 12'. Sie sind so hintereinander angeordnet, dass sie während des Haltens der Messschablone 2 zwangsläufig mit Daumen-Zeigefinger-Griff in Kontakt kommen (In der Zeichnungsfigur 6 ist nur die Ausgangselektrode 12' sichtbar).

Die Unterseite 5 der Messschablone 2 ist im Bereich der mittleren Schablonenabschnitte 6, 7 links und rechts von der Schablonenmitte jeweils mit einer Eingangselektrode 13 und einer Ausgangselektrode 14 versehen. Die Ausgangselektroden 14 sind links und rechts 7 cm und die Eingangselektroden 13 links und rechts 11 cm von der mit einer Markierung 11 versehenen Schablonenmitte entfernt angeordnet (in der Zeichnungsfigur 6 sind die Elektrodenpaare 13 und 14 auf der Unterseite als schraffierte Felder mit gestrichelter Umrand dargestellt, da sie aus dieser Perspektive normal nicht sichtbar wären).

Die Markierung 11 der Schablonenmitte dient als Hilfsmittel, um vor der Messung die Schablonenmitte auf den Bauchnabel (21, Fig. 1) positionieren zu können.

Im dargestellten Ausführungsbeispiel sind keine Elektrodenverbindungsleitungen eingezeichnet, da diese in die Messschablone integriert sind und in eine an der Oberseite 4 der Messschablone 2 angeordnete Kontaktstiftleiste 10 münden. Die zur Messung erforderlichen Stromimpulserzeuger und Spannungsabfallmesser sind gleichfalls nicht eingezeichnet, da sie Bestandteile des Messmoduls 1 mit Display und Bedientasten 1' sind, welches in die Kontaktstiftleiste 10 einschiebbar ausgebildet ist.

### Bezugszeichenliste

- 1: Messmodul mit Display
- 1': Bedientasten
- 2: Messschablone
- 3: bandförmiger Grundkörper der Messschablone
- 4: Oberseite des Grundkörpers
- 5: Unterseite des Grundkörpers
- 6, 7: mittlere Schablonenabschnitte
- 8, 9: Handgriffe der Messschablone
- 10: Kontaktstiftleiste
- 11: Markierung
- 12: Eingangselektroden im Bereich des Zeigefingers
- 12': Ausgangselektroden im Bereich des Daumens
- 13: Eingangselektrode in den mittleren Schablonenabschnitten
- 14: Ausgangselektrode in den mittleren Schablonenabschnitten
- 15, 16: äußere Schablonenabschnitte
- 17: Stromimpulserzeuger
- 18: Spannungsabfallmesser
- 19: Elektrodenverbindungskabel
- 20: elektromagnetische Felder
- 20': elektromagnetische Feldlinien
- 21: Bauchnabel
- 22: Richtungspfeil der möglichen Biegung
- 23: Messbereich

## Patentansprüche

1. Verfahren zur bioelektrischen Impedanz Messung (BIA) des Körpers einer Person, wobei mehrere einen Stromimpuls gebende Eingangselektroden (12, 13) und mehrere den Spannungsabfall messende sensitive Ausgangselektroden (12', 14 mit der Oberfläche des Körpers in Kontakt gebracht und zwischen diesen Elektroden (12, 12', 13, 14) die bioelektrische Impedanz der Körperabschnitte messtechnisch erfasst und die erhaltenen Messwerte mit gespeicherten, beispielweise statistischen Daten zu einer Körperstruktur- und viszeralen Risikoanalyse umgerechnet werden, **dadurch gekennzeichnet, dass** mittels einer bandförmig ausgebildeten, mit einem Messmodul (1) verbundenen Messschablone (2), die in den beiden mittleren Schablonenabschnitten (6, 7) jeweils links und rechts eine Eingangselektrode (13) und eine Ausgangselektrode (14) und an den Handgriffen (8, 9) der äußeren Schablonenabschnitte (15, 16) jeweils links und rechts eine Eingangselektrode (12) und eine Ausgangselektrode (12') aufweist, von der Person autark in einfacher Weise mit reproduzierbarer Genauigkeit gleichzeitig eine BIA-Bauch, eine BIA-Bauch-Hand und eine BIA-Hand-Hand durchgeführt wird, wozu die Messschablone (2) von der Person an den Handgriffen (8, 9) - mit Daumen-Zeigefingerkontakt an den dortigen Elektroden (12, 12') - gegriffen und ihre mittleren Schablonenabschnitte (6, 7) mit den Elektroden (13, 14) in definierter Position gegen den Bauch gehalten werden.

2. Verfahren nach Anspruch 1, **dadurch gekennzeichnet, dass** zur Sicherstellung der definierten Position bei der Messung die Messschablone (2) mit einer vorgegebenen Armhaltung gehalten und mit ihrer Mitte am Bauchnabel (21) ausgerichtet ist.

3. Verfahren nach Anspruch 1 oder 2, **dadurch gekennzeichnet, dass** die Messung mit einer Messfrequenz von 50 kHz Wechselstromimpulsen und ohne zusätzliche Befestigung oder Verkabelung am Körper durchgeführt und die im Messmodul (1) erhaltenen Messwerte mit einer integrierten Computereinheit bzw. Berechnungseinheit zur Erstellung einer Körperstruktur- und viszeralen Risikoanalyse verwendet werden.

4. Verfahren nach Anspruch 3, **dadurch gekennzeichnet, dass** die Messung mit einer weiteren und/oder mehreren Messfrequenzen zwischen 1-1000 kHz Wechselstromimpulsen durchgeführt werden.

5. Vorrichtung zur Durchführung des Verfahrens nach einem oder mehreren der Ansprüche 1 bis 4, beinhaltend:
• eine mit einem Messmodul (1) verbundene Messschablone (2), deren bandförmiger Grundkörper (3) so ergonomisch geformt ist, dass die Messschablone (2) mit ihren an den äußeren Schablonenabschnitten (15, 16) angeordneten Handgriffen (8, 9) quasi in nur einer vorgegebenen Armhaltung passend an den Bauch gehalten bzw. gegen diesen gedrückt werden kann;
• in den Handgriffen (8, 9) angeordnete Elektroden (12, 12'), wobei beim Halten der Messschablone (2) zwangsläufig jeweils der Daumen mit der Ausgangselektrode (12') und der Zeigefinger mit der Eingangselektrode (12) in Kontakt gebracht wird;
• an der Unterseite (5) der Messschablone (2) im Bereich der mittleren Schablonenabschnitte (6, 7) Jeweils links und rechts von der Schablonenmitte entfernt angeordnet eine Eingangselektrode (13) und eine Ausgangselektrode (14), die beim Halten der Messschablone (2) zwangsläufig am Bauch gehalten werden.

6. Vorrichtung nach Anspruch 5, **dadurch gekennzeichnet, dass** zur richtigen Positionierung der Schablonenmitte auf den Bauchnabel (21) die Schablonenmitte durch eine Markierung (11), beispielsweise eine Aussparung gekennzeichnet ist.

7. Vorrichtung nach Anspruch 6, **dadurch gekennzeichnet, dass** die Bauchelektroden (13, 14) an der Unterseite (5) der Messschablone (2) symmetrisch so um die Markierung (11) angeordnet sind, so dass sich die Ausgangselektroden (14) beispielsweise links und rechts 7 cm und weiter außen sich die Eingangselektroden beispielsweise links und rechts 11 cm entfernt von der Markierung (11) befinden.

8. Vorrichtung nach Anspruch 5, 6 oder 7, **dadurch gekennzeichnet, dass** die äußeren Schablonenabschnitte (15, 16) des bandförmigen Grundkörpers (3) so elastisch ausgebildet sind, dass sie sich während des Messvorgangs beim Halten der Messschablone (2) dem Bauchumfang anpassen.

9. Vorrichtung nach Anspruch 5, 6, 7 oder 8, **dadurch gekennzeichnet, dass** das mit der Messschablone (2) verbundene Messmodul (1) einen Stromimpulserzeuger (17) für die Eingangselektroden (12, 13) und einen Spannungsabfallmesser (18) für die Ausgangselektroden (14, 12') enthält und mit einer integrierten Computereinheit bzw. Berechnungseinheit zur Erstellung einer Körperstruktur- und viszeralen Risikoanalyse ausgestattet ist.

10. Vorrichtung nach Anspruch 9, **dadurch gekennzeichnet, dass** das Messmodul (1) sowie die Elektrodenverbindungsleitung als fester Bestandteil in die Messschablone (2) integriert sind.

11. Vorrichtung nach Anspruch 9, **dadurch gekennzeichnet, dass** das Messmodul (1) in eine an der Oberseite (4) der Messschablone (2) angeordnete Kontaktstiftleiste (10), in die die Verbindungsleitung (19) der Elektroden einmünden, eingeschoben ist.

12. Vorrichtung nach Anspruch 9, **dadurch gekennzeichnet, dass** das Messmodul (1) über eine die Elektroden-Verbindungsleitung (19) enthaltende gemeinsame Leitung mit der Messschablone (2) verbunden ist.

13. Vorrichtung nach Anspruch 10, 11 oder 12, **dadurch gekennzeichnet, dass** das Messmodul (1) die Messdaten mit einem integrierten Funkmodul an eine externe Station übermittelt.

## Claims

1. A method for bioelectric impedance analysis (BIA) of the body of a person, wherein a plurality of input electrodes (12, 13) emitting current pulses and a plurality of sensitive output electrodes (12', 14) measuring a voltage drop are brought in contact with the surface of the body, and between these electrodes (12, 12', 13, 14) the bioelectrical impedance of the body portions is metrologically detected, and the obtained measuring values are converted to a body-structure and visceral risk analysis with the aid of stored, e.g. statistical, data,
**characterized in that**
by means of a ribbon-like measuring template (2) connected to a measuring module (1) and comprising, in the two middle template portions (6, 7), an input electrode (13) and an output electrode (14) on the left-hand side and the right-hand side, respectively, and at the handles (8, 9) of the outer template portions (15, 16) an input electrode (12) and an output electrode (12') on the left-hand side and the right-hand side, respectively, a person can simultaneously carry out in an autarkical and simple manner and with reproducible accuracy a BIA-abdomen, a BIA-abdomen-hand and a BIA-hand-hand, for which purpose the measuring template (2) is grasped by the person at the grips (8, 9) - with thumb-index finger contact at the electrodes (12, 12') located there - and its middle template portions (6, 7) with the electrodes (13, 14) are held against the abdomen in a defined position.

2. The method according to claim 1, **characterized in that** for ensuring the defined position, during the measurement the measuring template (2) is held with a predetermined arm posture and its middle is oriented at the navel (21).

3. The method according to claim 1 or 2, **characterized in that** the measurement is carried out at a measuring frequency of 50 kHz alternating-current pulses and without any additional fastening or cabling at the body, and the measuring values obtained in the measuring module (1) are used with an integrated computer unit or calculating unit for establishing a body-structure and visceral risk analysis.

4. The method according to claim 3, **characterized in that** the measurement is carried out at another and/or a plurality of measuring frequencies between 1-1000 kHz alternating-current pulses.

5. A device for carrying out the method according to any one or several of claims 1 to 4, comprising:
• a measuring template (2) connected to a measuring module (1), whose ribbon-like base body (3) is ergonomically shaped such that the measuring template (2), with its grips (8, 9) arranged at its outer template portions (15, 16), can quasi only with one predetermined arm posture be fittingly held against the abdomen or pressed against the latter;
• electrodes (12, 12') arranged in the grips (8, 9), wherein, when the measuring template (2) is held, inevitably the thumb is brought into contact with the output electrode (12') and the index finger is brought into contact with the input electrode (12);
• on the lower side (5) of the measuring template (2) in the area of the middle template portions (6, 7) an input electrode (13) and an output electrode (14) are arranged at a distance to the template middle (14) to the left and to the right, respectively, thereof, which electrodes are inevitably held to the abdomen when holding the measuring template (2).

6. The device according to claim 5, **characterized in that** for proper positioning of the template middle on the navel (21), the template middle is marked by a marking (11), e.g. a recess.

7. The device according to claim 6, **characterized in that** the abdomen electrodes (13, 14), on the lower side (5) of the measuring template (2), are symmetrically arranged relative to the marking (11) such that the output electrodes (14) are e.g. located to the left and to the right at a distance of 7 cm, and further outside the input electrodes are arranged to the left and to the right e.g. at a distance of 11 cm to the marking (11).

8. The device according to claim 5, 6 or 7, **characterized in that** the outer template portions (15, 16) of the ribbon-like base body (3) are elastically formed such that they adapt to the abdominal girth when the measuring template (2) is held during the measuring process.

9. The device according to claim 5, 6, 7 or 8, **characterized in that** the measuring module (1) connected to the measuring template (2) includes a current pulse generator (17) for the input electrodes (12, 13) and a voltage drop meter (18) for the output electrodes (14, 12') and is provided with an integrated computer unit or a calculating unit for performing a body-structure and visceral risk analysis.

10. The device according to claim 9, **characterized in that** the measuring module (1) as well as the electrode connecting line are integrated as an integral part in the measuring template (2).

11. The device according to claim 9, **characterized in that** the measuring module (1) is inserted into a contact pin strip (10) arranged on the upper side (4) of the measuring template (2), into which strip the connecting line (19) of the electrodes lead.

12. The device according to claim 9, **characterized in that** the measuring module (1) is connected to the measuring template (2) via a common line including the electrode connecting line (19).

13. The device according to claim 10, 11 or 12, **characterized in that** the measuring module (1) transmits the measuring data to an external station by means of an integrated radio module.

## Revendications

1. Procédé de mesure d'impédance bioélectrique (BIA) du corps d'une personne, plusieurs électrodes d'entrée (12, 13) donnant une impulsion de courant et plusieurs électrodes de sortie sensitives (12', 14) mesurant la chute de tension étant mis en contact avec la surface du corps et l'impédance bioélectrique des parties de corps étant mesurée entre ces électrodes (12, 12', 13, 14) et les valeurs de mesure obtenues étant converties, à l'aide de données mémorisées, par exemple statistiques, en une analyse de risque viscéral et de structure du corps, **caractérisé en ce qu'**il est effectué par la personne, de manière autonome et de façon simple, avec une précision reproductible, à l'aide d'un calibre de mesure (2) ayant la forme d'une bande et étant relié à un module de mesure (1) et qui comprend dans les deux parties centrales (6, 7) du calibre respectivement à gauche et à droite une électrode d'entrée (13) et une électrode de sortie (14) et sur les poignées (8, 9) des parties extérieures (15, 16) du calibre respectivement à gauche et à droite une électrode d'entrée (12) et une électrode de sortie (12'), une BIA du ventre, une BIA ventre-main et une BIA main-main, le calibre de mesure (2) étant saisi par la personne aux poignées (8, 9) - avec un contact pouce-index sur les électrodes (12, 12') qui s'y trouvent - et les parties centrales (6, 7) du calibre étant tenues avec les électrodes (13, 14) dans une position définie contre le ventre.

2. Procédé selon la revendication 1, **caractérisé en ce que**, pour assurer la position définie lors de l'action de mesurer, le calibre de mesure (2) est tenu avec une position du bras prédéterminée et que son centre est aligné sur le nombril (21).

3. Procédé selon la revendication 1 ou 2, **caractérisé en ce que** l'action de mesurer est effectuée avec des impulsions de courant alternatif ayant une fréquence de mesure de 50 Hz et sans fixation supplémentaire ou câblage sur le corps, et **en ce que** les valeurs de mesure obtenues dans le module de mesure (1) sont utilisées avec une unité d'ordinateur ou unité de calcul intégrée pour établir une analyse de risque viscéral et de structure du corps.

4. Procédé selon la revendication 3, **caractérisé en ce que** l'action de mesure est effectuée avec des impulsions de courant alternatif ayant une autre et/ou plusieurs fréquences de mesure entre 1 et 1000 kHz.

5. Dispositif pour la mise en oeuvre du procédé selon l'une ou plusieurs des revendications 1 à 4, comprenant :
- un calibre de mesure (2) relié à un module de mesure (1), dont le corps de base (3) ayant la forme d'une bande est formé ergonomiquement de façon telle que le calibre de mesure (2) ne puisse être tenu sur le ventre ou pressé contre celui-ci, par ses poignées (8, 9) disposées sur ses parties de calibre extérieures (15, 16), quasiment que par une seule position de bras prédéterminé ;
- des électrodes (12, 12') disposées dans les poignées (8, 9), où, lors de la tenue du calibre de mesure (2), le pouce est forcément mis en contact avec l'électrode de sortie (12') et l'index avec l'électrode d'entrée (12) ;
- sur la face inférieure (5) du calibre de mesure (2), dans la zone des parties centrales (6, 7) du calibre, une électrode d'entrée (13) et une électrode de sortie (14) disposées respectivement à gauche et à droite du centre du calibre de mesure, lesquelles sont tenues forcément contre le ventre lorsque le calibre de mesure (2) est tenu.

6. Dispositif selon la revendication 5, **caractérisé en ce que**, pour un positionnement correct du centre du calibre sur le nombril (21), le centre du calibre est marqué par un marquage (11), par exemple un évidement.

7. Dispositif selon la revendication 6, **caractérisé en ce que** les électrodes de ventre (13, 14) sont disposées sur la face inférieure (5) du calibre de mesure (2) symétriquement par rapport au marquage (11) de façon telle que les électrodes de sortie (14) se trouvent par exemple à gauche et à droite à une distance de 7 cm du marquage (11) et les électrodes d'entrée par exemple à gauche et à droite à une distance de 11 cm.

8. Dispositif selon la revendication 5, 6 ou 7, **caractérisé en ce que** les parties de calibre extérieures (15, 16) du corps de base (3) ayant la forme d'une bande, sont formées élastiquement de façon telle qu'elles s'adaptent, lors de l'action de mesurer quand le calibre de mesure (2) est tenu, au tour de ventre.

9. Dispositif selon la revendication 5, 6, 7 ou 8, **caractérisé en ce que** le module de mesure (1) relié au calibre de mesure (2) comprend un générateur d'impulsions (17) pour les électrodes d'entrée (12, 13) et un dispositif de mesure (18) pour mesurer la chute de tension pour les électrodes de sortie (14, 12') et **en ce qu'**il est équipé d'une unité d'ordinateur ou de calcul intégrée pour établir une analyse de risque viscéral et de structure de corps.

10. Dispositif selon la revendication 9, **caractérisé en ce que** le module de mesure (1) ainsi que la ligne de liaison des électrodes sont intégrés dans le calibre de mesure (2) comme une partie fixe.

11. Dispositif selon la revendication 9, **caractérisé en ce que** le module de mesure (1) est inséré dans une bande de broches de contact (10) disposée sur la face supérieure (4) du calibre de mesure (2) dans laquelle aboutit la ligne de liaison (19) des électrodes.

12. Dispositif selon la revendication 9, **caractérisé en ce que** le module de mesure (1) est relié au calibre de mesure (2) par une ligne commune comprenant la ligne de liaison (19) des électrodes.

13. Dispositif selon la revendication 10, 11 ou 12, **caractérisé en ce que** le module de mesure (1) transmet les valeurs de mesure, à l'aide d'un module radio intégré, à une station externe.
